# EUROPÄISCHE PATENTANMELDUNG

(11) **EP 0 844 007 A2**
(43) Veröffentlichungstag der Anmeldung: **27.05.1998**
(21) Anmeldenummer: 97118019.5
(22) Anmeldetag: 17.10.1997
(51) Int. Cl.: A61M 15/00

(54) **Spender für Medien**

(30) Priorität: 20.11.1996 DE 19647947
(71) Anmelder: Ing. Erich Pfeiffer GmbH, 78315 Radolfzell (DE)
(72) Erfinder: Amann, Esther, 78315 Radolfzell (DE); Fuchs, Karl-Heinz, 78315 Radolfzell (DE)
(74) Vertreter: Patentanwälte Ruff, Beier, Schöndorf und Mütschele

(57) **Zusammenfassung**

Zur Inhalation weist der Spender (1) im Anschluß an einen Dosisspeicher (17) eine Einrichtung (20) zur Verwirbelung und Aufschließung des Mediums auf, das von einem den Speicher (17) durchströmenden Luftstrom mitgenommen und dann durch einen schräg gestellten Auslaß (12) abgegeben wird. Nach Öffnen des Speichers (17) kann das Medium in eine Vorlage (21) rieseln und von dort dann mit dem Luftstrom durch einen verengten Übertritt (25) mitgerissen werden.

## Beschreibung

Die Erfindung betrifft einen Spender für Medien, die gasförmig, flüssig, pastös und/oder pulverförmig sein können. Der Spender ist zweckmäßig mit einer einzigen Hand frei zu tragen und gleichzeitig mit dieser Hand zu betätigen bzw. anzuwenden. Im wesentlichen alle Teile, insbesondere Gehäuseteile, können aus Kunststoff bzw. Spritzguß bestehen, so daß ihre Wandungsdicke nicht mehr als 5 mm oder 2 mm beträgt. Das Medium soll insbesondere fein verteilt in einem Fluidstrom, wie einem Gas oder Luft gefördert und in möglichst genau dosierten Einzelquanten ausgetragen und hierfür innerhalb der Vorrichtung durch mehrfache Umlenkung ausreichend verwirbelt werden.

Soll der Spender zum Inhalieren eines pharmazeutischen Mediums dienen, so wird dieses zweckmäßig dem Förderstrom erst während der Anwendung beigemischt, wobei es zuvor wesentlich stärker verdichtet bzw. kompakt gespeichert ist.

Der Erfindung liegt die Aufgabe zugrunde, einen Spender zu schaffen, bei welchem Nachteile bekannter Ausbildungen vermieden bzw. Vorteile der genannten Art zu erzielen sind und der insbesondere ein genau dosiertes, tief bis ans innere Ende der Atemwege eines Patienten reichendes Einbringen des Mediums in feinster Zerstäubung bei ergonomisch günstiger Handhabung gewährleistet.

Erfindungsgemäß sind Mittel vorgesehen, um das Medium innerhalb der Förderwege des Spenders sehr fein aufzuschließen, beispielsweise durch nur ein- oder mehrfach hin- und hergehende Bewegung des Mediums. So können bereits vorhandene größere Partikel in kleinere Partikel an mindestens zwei einander gegenüberliegenden Prallflächen separiert werden. Im Falle eines Pulvers kann dieses mit oder ohne Lufstrom vom Speicher zunächst nach unten in eine muldenförmige Prall- oder Leitfläche gelangen. Danach wird es durch den Luftstrom von dieser ersten Fläche mit hoher Geschwindigkeit abgehoben, verwirbelt und an eine gegenüberliegende Wandung geschleudert. Das führt zu einer Zerkleinerung der eventuell verklumpten Pulverteilchen. Der Anteil der lungengängigen, d.h. bis in die Lunge des Patienten vordringenden Partikel wird dadurch gegenüber solchen Spendern wesentlich gesteigert, welche lediglich zur nasalen Applikation oder zur Anwendung des Mediums im Rachenraum vorgesehen sind.

Die genannte erste oder eine andere Fläche kann auch als Zwischenspeicher oder Vorlage für wenigstens einen Teil der Einzel-Anwendungsdosis des Mediums vorgesehen sein. Wenigstens ein Teil dieser Dosis des fließfähigen Mediums fällt dann während des Öffnens und ggf. danach auf die Vorlage, die rinnenförmig nach oben erweitert ist. Mit dem erst danach einsetzenden Förderstrom wird dieses Medium von der Vorlage unter Verwirbelung abgehoben. Sofort danach wird es gegen die darüberliegende Wandung geschleudert und an dieser wieder in die Fallrichtung zum Auslaß oder Mundstück umgelenkt.

Der Förderstrom durchströmt zweckmäßig durch geeignete Abdichtung vollständig den Speicherraum. So werden im Speicher verbliebene Rückstände des Mediums bis zum Auslaß mitgenommen. Auch diese Rückstände gelangen vom Speicher berührungsfrei unmittelbar auf die Vorlage. Diese kann geneigte Rutschflächen aufweisen. Dadurch kann das Medium unter seiner Gewichtskraft auch ohne Förderstrom bis an die tiefste Stelle der Vorlage gelangen.

Zwischem dem Speicherauslaß und der Vorlage kann noch ein Teilungsglied zur Auffächerung des Medienstromes vorgesehen sein, beispielsweise ein Dorn oder eine Spitze. Diese dient auch zum Öffnen des Speichers. Die Spitze ragt bis in den Speicherraum bzw. das in diesem enthaltene Medium hinein.

Vorteilhaft ist der Förderweg zwischen dem Speicher und dem Auslaß, an welchem das Medium unter vollständiger Ablösung von der Spender ins Freie austritt, möglichst kurz. Ab der Verwirbelungszone ist der Förderweg möglichst geradlinig oder nur ein einziges Mal stumpfwinklig abgewinkelt bzw. gekrümmt. So entstehen geringe Strömungsverluste. Der erste Weg zwischen dem Speicherauslaß und der Verwirbelungszone ist kürzer als der zweite Strömungsweg zwischen der Mitte dieser Verwirbelungszone und dem Auslaß. Der zweite Weg ist höchstens vier- oder dreimal länger als der erste Weg. Die Mitte der Verwirbelungszone kann mit der Mitte der gegenüberliegenden Prallfläche zusammenfallen. Der geringste Durchlaßquerschnitt der Förderwege ist vorteilhaft kleiner als der durchgehend konstante Durchlaßquerschnitt des Speicherraumes und liegt vorteilhaft an dem Übertritt. Dieser wird als engste Stelle von den beiden einander gegenüberliegenden Flächen begrenzt. Diese leiten das Medium von der Vorlage in den zum Auslaß führenden Endkanal. Letzterer kann durchgehend konstante Durchlaßquerschnitte haben.

Der geradlinige Endkanal bildet mit einem Ende den Auslaß. Dieser und der Endkanal liegen unter einem Winkel zur Speicher- bzw. Hauptachse der Vorrichtung. Diese kann daher bei oraler Anwendung mit leicht zurückgeneigtem Kopf annähernd vertikal gehalten werden. Der Daumen der Bedienungsperson liegt dann zwischen Oberlippe und Mundstück und kann an beide angelegt werden.

In den Förderwegen kann stromaufwärts und/oder stromabwärts vom Speicherraum ein druckabhängig öffnendes Ventil vorgesehen sein. Erst nach Erreichen eines vorbestimmten Unterdruckes stromabwärts vom Ventil wird der Förderstrom durch die Ventilöffnung schlagartig in Gang gesetzt. Es ergeben sich sehr hohe Strömungsgeschwindigkeiten. Das Ventil kann ein Manschettenventil sein.

Diese und weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein können. Ausführungsbeispiele der Erfindung sind in den Zeichnungen dargestellt und werden im folgenden näher erläutert. In den Zeichnungen zeigen:
- Fig. 1: ein erfindungsgemäßer Spender im Axialschnitt,
- Fig. 2: der Spender gemäß Fig. 1 in teilweise geschnittener Ansicht von unten und
- Fig. 3: einen Ausschnitt einer weiteren Ausführungsform mit Druckerzeuger für einen Förderstrom.

Der Spender 1 weist einen Grundkörper 2 aus nur fünf Gehäuseteilen 3 bis 7 auf. Von diesen bilden im Bereitschafts- bzw. Betriebszustand lediglich drei festsitzend verbundene Teile 3, 4, 6 gemeinsam mit einer Magazin-Einheit 8 die gesamte Außenfläche der Austrag-Vorrichtung 1. Der Teil 5 ist vollständig versenkt festsitzend im Teil 3 angeordnet und durch den Teil 4 unmittelbar axial gesichert. Die Einheit 8 weist mindestens vier und höchsten acht gleichmäßig verteilte und unmittelbar benachbart zueinander in einem Kranz um eine Achse 10 angeordnete Speicherplätze 9 für das Medium auf. Die Achse 10 ist parallel zur Hauptachse 11, in welcher der jeweils in Betriebstellung stehende Platz 9 liegt. Unmittelbar aus dieser Stellung heraus wird der Platz 9 für den Austrag durch einen Auslaß 12 entleert. Dessen Achse 13 liegt unter einem stumpfen Winkel von mindestens 110° und höchstens 160°, insbesondere 135°, zur Achse 10 bzw. 11. In Ansicht parallel zur Achse 10, 11 liegt der Auslaß 12 vollständig innerhalb des Außenumfanges des Grundkörpers 2.

Vollständig innerhalb des Grundkörpers 2 ist eine dessen beide Enden verbindende Fluidführung 14 bzw. Kanalisierung vorgesehen. Zwischen deren Enden liegt ein Speicherauslaß 15, um das Medium abzugeben. Der Auslaß 15 hat einen wesentlich kleineren Abstand vom stromaufwärts liegenden Ende der Führung 14 als von deren Austrittsende 12. Der Auslaß 15 ist durch ein Ende eines langgestreckten, gesonderten Speicherkörpers 16 mit langgestrecktem Speicherraum 17 gebildet. Dieser ist in der Entleerungsstellung mit der Achse 11 koaxial. Der formsteife Körper 16 ist durch eine zweiteilige Kapsel aus Hartgelatine o. dgl. gebildet. Deren beide hülsenförmige Teile sind eng aneinander angepaßt axial zusammengesteckt. Die voneinander abgekehrten Kapselenden sind halbkugelförmig. Das im Raum 17 enthaltene Medium ist vor Öffnung mit einer Vorrichtung 18 dicht verschlossen verpackt. Das nicht gasförmige, fließfähige Medium füllt den Raum 17 vollständig oder nur teilweise als Einzel-Anwendungsdosis aus. Der Körper 16 bzw. Raum 17 ist nur durch Zerstörung zu öffnen. Er bildet im Betrieb einen über seine gesamte Länge reichenden Abschnitt der Führung 14. Die an den beiden Raumenden liegenden Öffnungen sind drosselartig wesentlich enger als zwischen den Enden liegende Teil des Raumes 17. Dieser Teil hat durchgehend konstante Durchlaßquerschnitte.

Der freiliegende, nur durch die Teile 3, 4, 6, 8 gebildete Außenmantel 19 der Vorrichtung 1 kann mit einer einzigen Hand nahezu vollständig umschlossen werden. Innerhalb dieses Mantels 19 bildet die Führung 14 eine Zone 20 zur Verwirbelung, Zerkleinerung und feinst verteilten Aufbereitung des Mediums. Das Medium wurde bereits stromaufwärts der Zone 20 vom Luftstrom aufgenommen. Mittig zwischen den Enden der Führung 14 bzw. der Vorrichtung 1 ist in Betriebsstellung unterhalb des Auslasses 15 eine schalen- oder rinnenförmige Vorlage 21 vorgesehen. Sie ist nur nach oben offen. Ihr Boden 24 hat vom Auslaß 15 einen gegenüber der Länge des Raumes 17 kleineren Abstand. Der konkav gekrümmte Boden 24 schließt an eine längere Flanke 22 und eine kürzere Flanke 23 an. Die Flanken 22, 23 divergieren nach oben spitzwinklig. Die zwischen den Achsen 10, 11, jedoch näher bei der Achse 10 liegende gemeinsame Axialebene der Flächen 22 bis 24 bzw. der Mulde 21 ist seitlich gegenüber der Achse 11 des Auslasses 15 versetzt. Der Auslaß 15 liegt vertikal über der Mitte des Bodens 24, wenn die Vorrichtung 1 in Betriebstellung geringfügig nach hinten gekippt ist. Dabei ist die Achse 13 flacher als bei vertikaler Ausrichtung der Achsen 10, 11 schräg gestellt ist.

Die Flanke 22 schließt abgedichtet an den Auslaß 15 an. Die Flanke 23 reicht nur bis zu einem verengten Übertritt 25 zwischen der Mulde 21 und dem stromabwärts davon liegenden Teil der Führung 14. Die Flanke 23 reicht bis zu einer abgerundeten Kante 26, die über ihre Länge konkav ist. Die Kante 26 liegt einer darüberliegenden, konkaven Fläche 27 gegenüber. Sie ist wie die Mulde 21 um eine zu den Achsen 10, 11 rechtwinklig querliegende Achse gekrümmt. Sie hat einen gegenüber dem Boden 24 mindestens vier- oder fünfach größeren Krümmungsradius. Die Fläche 27 reicht in und entgegen Strömungsrichtung über den von der Kante 26 begrenzten Übertritt 25 hinaus. Die Fläche 27 reicht bis zum Auslaß 15 und als Umfangsbegrenzung bis in einen Kanalabschnitt 28. Dieser schließt stromabwärts an den Übertritt 25 und die Kante 26 an.

Das stromabwärts liegende Ende des Abschnittes 28 schließt stumpfwinklig gekrümmt an einen Endkanal 29 an. Dessen Ende bildet den Auslaß 12. Die Achse 30 des Abschnittes 28 liegt parallel zur Achse 10, 11 und auf der von der Achse 11 abgekehrten Seite der Achse 10. Die beiden Kanalabschnitte 28, 29 sind jeweils geradlinig. Sie haben durchgehend konstante Durchlaßquerschnitte, welche größer als derjenige des Übertrittes 25 sind. Der Abschnitt 29 ist durch einen frei vorstehenden, rohrförmigen Stutzen 31 konstanter Außenquerschnitte gebildet. Das Mundstück 31 ist über einen größten Teil seiner Länge in den Mund des Patienten einzuführen und mit dessen Lippen dicht zu umschließen. Der Abschnitt 29 kann dabei geringfügig länger als der Abschnitt 28 bis zur Kante 26 sein. Mulde 21 und Stutzen 31 liegen an derselben Seite des Abschnittes 28.

Die Mulde 21 einschließlich der Flächen 22 bis 24, 26 und eines ersten Längsabschnittes des Kanalteiles 28 ist ausschließlich vom Teil 5 begrenzt. Dieses ist entgegen Strömungsrichtung in den Teil 3 bis zum Anschlag vollständig versenkt eingesetzt und durch den in dieser Richtung ebenfalls eingesetzten Teil 4 axial gesichert. Der Teil 4 bildet einen innerhalb des Teiles 3 anschließenden Längsabschnitt des Kanalteiles 28 sowie den Abschnitt 29, den Stutzen 31 und den Auslaß 12. Der Teil 4 steht über den Außenumfang des Teiles 3 nicht vor und schlägt an dessen unterer, ringförmiger Stirnfläche mit einer Ringschulter abgedichtet an. Die Fläche 27 reicht über einen Bogenwinkel von weniger als 90° und mehr als 45°. Die Fläche 27 ist nur durch den Teil 3 gebildet sowie an beiden Enden kontinuierlich fortgesetzt. So bildet sie einen Zwischenabschnitt einer halbkreis- oder halkugelförmigen bzw. U-förmigen Innenfläche des Teiles 3. Diese Innenfläche weist an eine Fortsetzung auf, welche an die Fläche 27 anschließendt. Der Teil 5 liegt mit konvexen Flächen an dieser Fortsetzung dicht an. Im Anschluß an den Teil 5 liegt der Teil 4 mit einer Umfangsfläche dicht an der Fortsetzung an.

Die Flanke 22 reicht bis zu diesen gekrümmten Flächen. Die Fläche 27 ist im Bereich des Auslasses 15 sowie im Anschluß an die Flanke 22 von einer Übertrittsöffnung 46 für das Medium und den Lufstrom durchsetzt. Die Öffnung 46 liegt in der Achse 11. Der Durchlaßquerschnitt der Öffnung 46 entspricht dem größten Durchlaßquerschnitt des Raumes 17. Die Weite der Übertrittsöffnung 46 ist größer als die größte Weite des Raumes 17. Die Mulde 21 liegt zwischen den Achsen 11, 30. Die größte Weite der Mulde 21 ist in Höhe der Kante 26 größer als die zugehörige Tiefe dieser Mulde 21. Die Kanalabschnitte 21, 25, 28 weisen parallel zur Zeichenebene liegende Begrenzungen auf. Sie können annähernd eben sowie zueinander parallel sein. Dann ist die Vorrichtung im zugehörigen Bereich in Axialansicht stegförmig. Dieser Bereich kann wie die genannten Flächen, aber auch rotationsförmig um die Achse 11 gekrümmt sein.

Mit Abstand oberhalb der Anordnungen 12, 29, 31 bildet der Teil 4 des Körpers 2 eine ballig gekrümmte Handhabe 32. Sie schließt im einspringenden, spitzen Winkel rechtwinklig quer zur Achse 11 an das hintere Ende des Außenumfanges des Mundstückes 31 an. Zum davon entfernten Bereich steigt die Fläche 32 bis zum Außenumfang des Mantels 19 an. Die andere Handhabe 33 ist durch das hintere Ende des Körpers 2 gebildet, nämlich durch die Außenseite der Stirnwand des Teiles 6. Die beiden Handhaben bilden einen Handgriff 32, 33. Der Daumen kann an der Handhabe 32 anliegen und weitere Finger derselben Hand können klammerartig an der davon abgekehrten Handhabe 33 anliegen. Gleichzeitig ist das Mundstück 31 zwischen den Lippen des Patienten eingeführt. Dabei kann sich der Finger, welcher an der konvexen Handhabe 32 abgestützt ist, auch an der Oberlippe des Patienten sowie mit seiner davon abgekehrten Seite am Außenumfang des Mundstückes 31 anlegen. Die Handhaben 32, 33 liegen während des gesamten Betriebes und Austrages lagestarr zueinander.

Die Einheit 8 weist einen um die Achse 10 bewegbaren Magazinkörper 34 auf. Dieser ist axial zwischen den Teilen 3,6 festgelegt. Er trägt an seiner dem Teil 6 zugekehrten Seite auswechselbar einen Magazineinsatz 35 mit der genannten Anzahl der Speicherkörper 16. Der Körper 34 weist für jeden Platz 9 eine Halterung 36 auf, die in Strömungsrichtung frei vorsteht und hülsenförmig ist. Die Halterung 36 umschließt abgedichtet sowie eng nur das eine untere Ende des Körpers 16. Durch Verengung bildet der Mantel 36 einen Anschlag für die untere, gekrümmte Endfläche des Körpers 16. Eine entsprechende Halterung 37 steht nur in Strömungsrichtung vor. Sie ist wesentlich kürzer als die Halterung 36. Für jeden Platz 9 ist am Einsatz 35 ein Mantel 37 vorgesehen. Sie steht nur über die untere Stirnseite des Einsatzes 35 vor, der ansonsten kreis- bzw. scheibenförmig eben ist. Die Manschette 37, greift mit einem konischen Außenumfang in eine konische Innenfläche am hinteren Ende der Halterung 36 ein. So liegt sie radial verengt dicht am Außenumfang des engeren Teiles des Körpers 16 an. Dessen erweiterter Deckelteil kann mit seiner Stirnfläche an der oberen Stirnfläche der Einsatzscheibe 35 anliegen. Dadurch ragt dieses hintere Ende bzw. der Deckelteil entgegen Strömungsrichtung berührungsfrei in den Innenraum des Teiles 6. Der untere Längsabschnitt der Kapsel 16 liegt vollständig in den Halterungen 36, 37 und durchsetzt die Körper 34, 35. Wie jedes der anderen Teile 3 bis 7, 35 ist der Körper 34 einteilig ausgebildet. Er weist am äußersten Umfang einen Mantel 38 auf. An dessen Innenumfang schließt mit Abstand zwischen seinen Enden eine Stirnwand 39 an. Über deren Unterseite stehen die Halterungen 36 vor. An der oberen Stirnfläche liegt der Einsatz 35 an. Der Außenumfang des Mantels 38 bildet eine Handhabe 40. sie reicht um die Achse 10 über einen Bogenwinkel von mindestens 90° oder 160° und höchstens 220°, insbesondere nur 180°. Sie ist am Außenumfang der Körper 3, 6 nur über diesen Winkel zur Betätigung frei zugänglich. In der Betriebsstellung liegt das verengte Ende bzw. der Auslaß 15 der Halterung 36 unmittelbar benachbart zur Übertrittsöffnung 46 in der Fläche 27 bzw. zur Außenseite der gekrümmten Wandung 47, welche die Fläche 27 bildet.

Der Körper 34 liegt vollständig an dieser Außenseite. Er ist unmittelbar am Teil 3 mit zwei konzentrischen Lagern 41, 42 drehbar gelagert sowie in entgegengesetzter Richtung axial gesichert. Die Lagerteile sind einteilig mit dem Teil 3 ausgebildet. Sie sind durch zwei ineinander liegende Lagerkörper gebildet beispielsweise Hülsen, die entgegen der Strömungsrichtung frei ausragen. Mit ihren Endflächen gleiten die Hülsen an der Unterseite der Wand 39. Die äußere Hülse des äußeren Lagers 41 gleitet mit ihrem Außenumfang am Innenumfang des Mantels 38 und mit ihrem Innenumfang an den Außenumfängen der Halterungen 36. Die innere Hülse des inneren Lagers 42 gleitet mit ihrem Außenumfang ebenfalls an den Außenumfängen der Halterungen 36, die hierfür gemeinsam einen Innenumfang bilden. Zwischen den beiden Hülsen liegt die Öffnung 46, die an den Auslaß 15 anschließt. Die beiden Hülsen gehen einteilig in die gekrümmte Wandung der Fläche 27 über. Die Hülse des Lagers 41 ist exzentrisch zur Achse 11 des darunter anschließenden Gehäuseteiles 3, 4 des Körpers 2 vorgesehen und steht auf der von der Handhabe 32 abgekehrten Seite über die Teile 3, 4 vor. Zur Axialsicherung kann an einer der Hülsen, insbesondere zwischen dem Außenumfang der inneren Hülse und dem Körper 34, eine Schnappverbindung vorgesehen sein. Nach vollständigem Abnehmen des Teiles 6 ist der Einsatz 35 einschließlich der entleerten Körper 16 entgegen Strömungsrichtung herauszuziehen. Dabei muß der Körper 34 nicht aus der Lagerung 41, 42 gelöst werden.

Ein weiteres Radial- und Axiallager ist an der oberen Seite der Körper 35, 39 vorgesehen. Dafür gleitet der Mantel 43 des Teiles 6 auf dieser Seite am Innenumfang des Mantels 38 und an der oberen Stirnfläche des Körpers 35. Der Körper 35 wird in engem Kontakt mit der Oberseite der Wandung 39 gehalten. Der Mantel 43 bildet nur über einen Teilumfang auch den Außenmantel des Teiles 6, da er exzentrisch zu diesem Außenmantel liegt. Außerhalb des Lagergliedes 43 greift dieser Außenmantel in das Innere des Mantels des Teiles 3 festsitzend ein. Dabei ist der Außenmantel durch eine federnde Schnappsicherung gegen Abziehen gesichert. Durch Anwendung einer entsprechend hohen Abzugkraft kann er dennoch entgegen Strömungsrichtung abgelöst werden. Nach diesem Ablösen liegt der Körper 35 mit den Körpern 16 frei zugänglich zum Auswechseln.

Die Vorrichtung 18 weist zwei in der Achse 11 und einander gegenüberliegende Öffnungsglieder 44, 45 auf. Sie sind durch Metallspitzen gebildet und dienen zum Aufbrechen der Endwandungen der Kapsel 16 während der Schaltbewegung der Einheit 8. Die Kapsel 16 wird dadurch in der letzten Phase der Überführung in die Betriebsstellung von den Gliedern 44, 45 erfaßt und an den Enden zersplittert. Dann ragen die spitzen in den Raum 17 hinein, wobei jede von einer gezackten Öffnung umgeben ist. Die zugehörige Übertrittsöffnung 46 der gekrümmten Wand 47 wird vom Glied 44 durchsetzt. Es ist an der Wand 47 mit den Armen einer sternförmigen Halterung lagegesichert. Der Außenumfang der Spitze 44 bildet eine Leitfläche. Durch sie werden das Medium und der Luftstrom in einen Hüllstrom aufgeweitet. Die hintere Spitze 45 ist an der Innenseite der Stirnwand des Teiles 3 befestigt. Die Spitzen 44, 45 sind koaxial gegeneinander gerichtet. Der Ring 38, ist mit einer Anzeigeeinrichtung für seine Drehstellung versehen und rastet in jeder Betriebstellung federnd ein.

Zum Gebrauch des Spenders 1 wird der Ring 38 soweit gedreht, bis die nächste Kapsel 16 in der Achse 11 liegt und dann an beiden Enden geöffnet ist. Durch das Öffnen rieselt ein Teil des Mediums entlang der Spitze 44, dann über eine freie Fallstrecke und dann entlang der Flanke 22 bzw. 23 auf den Boden 24 der Mulde 21. Danach saugt der Patient am Mundstück 31. Dabei wird durch Öffnungen in dem Gehäuseraum, welcher das obere Ende der Kapsel 16 und die Spitze 45 aufnimmt, von außen Luft angesaugt. Sie strömt durch die obere Öffnung der Kapsel 16 in den Raum 17.

Die Luft durchströmt den Raum 17 unter Mitnahme des Restes des Mediums, das noch in diesem Raum 17 ist. Das Mischmedium strömt dann durch den Auslaß 15 um die Spitze 44 herum unmittelbar in die Öffnung 46. Von dieser strömt es gegen die näher beim Auslaß 12 liegende Flanke 22. Der Förderstrom wird entlang der Flanke 22 und des Bodens 24 zurück nach oben umgelenkt, reißt an der Kante 26 ab und wird unmittelbar gegen die Fläche 27 geleitet. Hierbei nimmt der Förderstrom das in der Mulde 21 befindliche Medium mit. Im Bereich der Mulde 21 kann dabei kurz eine Walzenströmung entstehen. Durch die Saugwirkung wird die Strömung beschleunigt. Der Förderstrom fließt durch den Übertritt 25 in den Kanal 28, 29. Dort erfolgt eine Strömungsberuhigung, die sich bis zum Auslaß 12 fortsetzt. Beim Auftreffen auf der Fläche 27, gegenüber welcher die Flanke 23 auf einem Radiusstrahl liegt, werden größere Medienpartikel durch Prallwirkung zerkleinert. Für die nächste Anwendung wird die Einheit 8 um einen Platz 9 weitergedreht. Die Einheit 8 ist mit einem Freilaufgesperre nur in einer einzigen Richtung drehbar.

Stromabwärts vom Auslaß 15 bzw. vom Übertritt 25 ist zweckmäßig im Kanal 28 ein Sieb 48 oder ein Filter vorgesehen. Es ist zwischen den Teilen 3, 4 eingesetzt. Dadurch können eventuell abgesplitterte Teilchen der Kapsel 16 oder zu große Medienpartikel nicht in den Rachenraum des Patienten gelangen. Ferner kann im Strömungsweg, nämlich stromaufwärts oder stromabwärts vom Raum 17, ein Ventil 49 vorgesehen sein. Es öffnet druckabhängig dann, wenn stromabwärts ein geringerer Druck als stromaufwärts anliegt. Das Ventil kann mit zunehmender Öffnung eine konstante oder abnehmende Öffnungskraft aufweisen. Dann öffnet es nach Beginn der Öffnung schlagartig vollständig und der Förderstrom wird impulsartig freigegeben. Das Ventil 49 kehrt druckabhängig zur Schließstellung zurück. Das Ventil 49 liegt nahe zum Auslaß 12 innerhalb des Kanales 29. Dadurch ist der stromaufwärts liegende Abschnitt der Führung 14 während der Ruhezeiten gegen Eindringen von Verunreinigungen dicht geschlossen. Der Teil 7 ist als Schutzkappe ausgebildet. Sie wird für den Gebrauch axial vollständig abgezogen. Der Teil 7 nimmt in Schutzlage den Stutzen 31 einschließlich Öffnung 12 sowie das gesamte Teil 4 und den unteren Abschnitt des Teiles 3 dicht verschlossen auf.

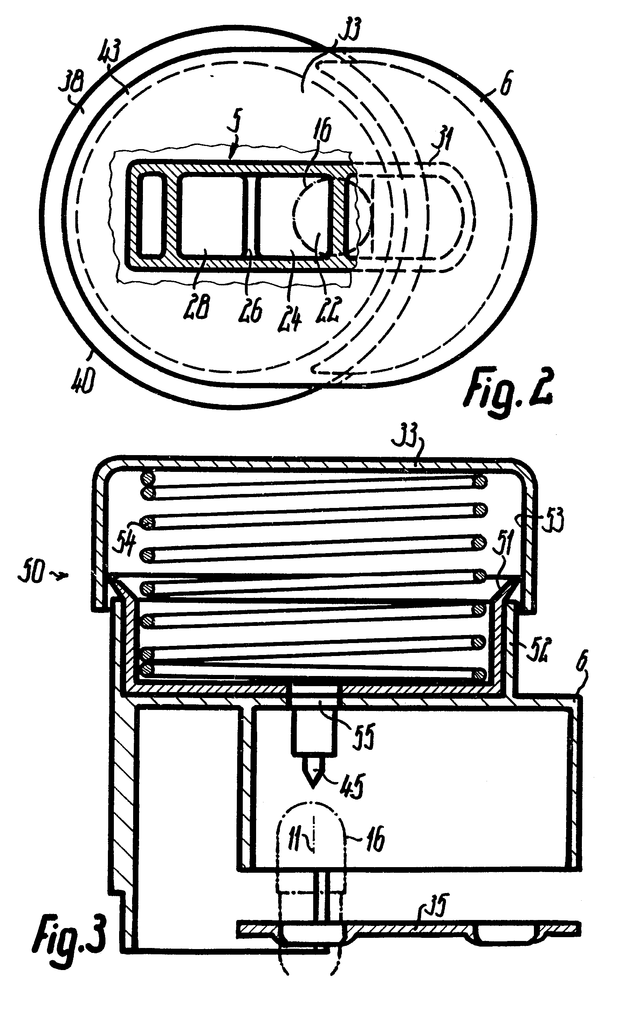

In Fig. 3 ist nur der obere Abschnitt des Spenders ab dem Teil 6 dargestellt. An dessen Oberseite ist als Druckerzeuger eine Luftpumpe 50 angeordnet. Die obere Stirnwand des Teiles 6 bildet hier nicht eine Handhabe, sondern weist einen nach oben frei vorstehenden Mantel 52 auf. In diesen ist ein napfförmiger Kolben 41 mit seinem Mantel bis zum Anschlag an der Stirnwand des Teiles 6 fest eingesetzt. Die erweiterte Kolbenlippe steht über das obere Ende des Mantels 52 vor. Die Kolbenlippe gleitet an einem Zylinder 53. Dieser umgibt den Außenumfang des Mantels 52 eng. Der Zylinder 53 kann bis zum Anschlag an der Stirnwand des Teiles 6 gegen die Kraft einer Feder 54 nach unten verschoben werden. Dadurch wird Luft durch eine Öffnung 55 im Kolbenboden sowie in der Stirnwand des Teiles 6 um die Spitze 45 herum der Kapsel 16 zugeführt. Die Pumpe 50 liegt in der Achse 11. Die Stirnwand des Zylinders 53 bildet die bewegliche Handhabe 33. Nach deren Freigabe kehrt die Pumpe unter Ansaugen von Frischluft in ihre Ausgangslage zurück. Dabei kann der Weg durch die Öffnung 55 mit einem Ventil, beispielsweise dem Ventil 49, gegen Ansaugen geschlossen sein. Auch hier kann der Luftstrom allein durch Ansaugen durch die Öffnung 12 erzeugt und zu jeder beliebigen Zeit durch Betätigen der Pumpe 50 verstärkt werden. In Fig. 3 ist der Einsatz 35 in seiner Wechselstellung für sich und ohne Einsatz 34 dargestellt.

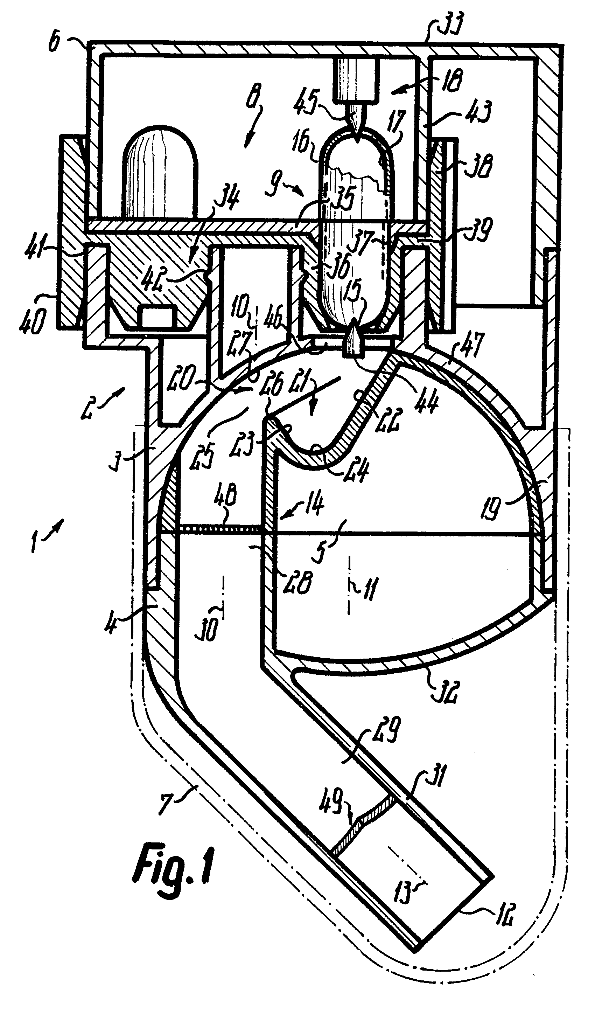

Alle angegebenen Wirkungen und Eigenschaften, wie Lagen, Größen usw., können genau wie beschrieben oder nur etwa bzw. im wesentlichen wie beschrieben vorgesehen sein und je nach Anwendungszweck auch stark davon abweichen. Der Spender kann maßstäblich gemäß den Figuren 1 bis 3 ausgebildet sein. Die Begrenzungsflächen der Bereiche, die mit dem Medium in Berührung kommen, insbesondere der Bereiche 12, 14, 18, 20 bis 29, 44 bis 46, 48 und 49, können mit einer antihaft- bzw. antistatischen Beschichtung aus Metall und/oder Kunststoff, wie Tetrafluoräthylen, versehen sein. Dadurch kann ein Anhaften des Mediums durch elektrische Aufladung od.dgl. vermieden werden. Die Beschichtung ist nur wenige µm dünn sein. Sie kann durch Lackieren, Kleben, Drucken od.dgl. auf die Flächen der genannten Bereiche aufgetragen sein.

## Patentansprüche

1. Spender für Medien, insbesondere Pulver od.dgl., dadurch gekennzeichnet, daß sie einen Grundkörper (2) und insbesondere eine Aufnahme mit wenigstens einem Speicherplatz (9) für das zu mindestens einer gesonderten EinzelAnwendungsdosis in einem Speicherraum (17) vorportionierte Medium aufweist und/oder daß durch Austragbetätigung in einem Bereitschaftszustand der Austragvorrichtung (1) die jeweilige Einzeldosis mit einem durch eine Fluidführung (14) strömendem Förderstrom durch einen Auslaß (12) aus der Austragvorrichtung (1) zu fördern ist, wobei vorzugsweise Mittel zur Übergabe und Ablage wenigstens eines Teiles der jeweils vorportionierten Einzeldosis an mindestens eine Zwischen-Vorlage (21) vorgesehen sind und/oder das Medium mit dem Förderstrom von der Vorlage (21) aufzunehmen ist.

2. Spender nach Anspruch 1, dadurch gekennzeichnet, daß dem Auslaß (12) Zerkleinerungsmittel (20) zur räumlich ausdehnenden Aufschließung des Mediums in Feinpartikel vorgeschaltet sind, daß insbesondere die Zerkleinerungsmittel (20) wenigstens teilweise in der Fluidführung (14) liegen und daß vorzugsweise die Zerkleinerungsmittel (20) wenigstens teilweise durch einen Strömungsbeschleuniger, eine Wirbelzone, eine Prallfläche (27) bzw. ein Sieb (48) gebildet sind.

3. Spender nach Anspruch 1 oder 2, dadurch gekennzeichnet, daß zwischen einem Speicherplatz (9) und der Vorlage (21) wenigstens ein Fallweg zur Überführung des Mediums zur Vorlage (21) unter Gewichtskraft vorgesehen ist, daß insbesondere der Fallweg eine berührungsfreie Fallstrecke bzw. eine abfallende Leitbahn (22, 23) aufweist und daß vorzugsweise der Fallweg ununterbrochen von einem Ausgang (15) des Speicherplatzes (9) bis zur Vorlage (21) durchgeht.

4. Spender nach einem der vorhergehenden Ansprüche, dadurch gekennzeichnet, daß die Vorlage (21) schalenförmig mit in Bereitschaftsstellung trichterförmig nach oben erweiterter Schalenöffnung bzw. konkav gekrümmtem Schalenboden (24) ausgebildet ist, daß insbesondere die Vorlage (21) an einer Seite (22) bis zu einer Abgabeöffnung (15, 46) für das Medium im wesentlichen vollständig geschlossen bzw. nur an einer Seite (23) über eine Übertrittsöffnung (25) an mindestens einen wegführenden Kanal (28, 29) der Fluidführung (14) angeschlossen ist und daß vorzugsweise die Vorlage (21) rinnenförmig mit unterschiedlich hohen Rinnenrändern (22, 23) ausgebildet ist.

5. Spender nach einem der vorhergehenden Ansprüche, dadurch gekennzeichnet, daß im Bereich der Vorlage (21) ein von deren Boden (24) zuerst aufsteigender und dann ein abfallender Kanalabschnitt (28) der Fluidführung (14) vorgesehen ist, daß insbesondere beide Kanalabschnitte einschließlich des Überganges (25) zwischen ihnen von einer der Vorlage (21) gegenüberliegenden und konkaven Leitfläche (27) begrenzt sind und daß vorzugsweise der abfallende Kanalabschnitt (28, 29) unter der Vorlage (21) schräg zum Auslaß (12) verläuft.

6. Spender nach einem der vorhergehenden Ansprüche, dadurch gekennzeichnet, daß der Speicherplatz (9) unmittelbar oberhalb der Vorlage (21) vorgesehen ist, daß insbesondere der Speicherraum (17) in Höhenrichtung langgestreckt ist und daß vorzugsweise der Speicherraum (17) im Bereitschaftszustand an beiden Enden geöffnet ist sowie einen Speicherkanal für den Förderstrom bildet.

7. Spender nach einem der vorhergehenden Ansprüche, dadurch gekennzeichnet, daß der Speicherraum (17) durch eine vollständig geschlossene Kapsel (16) gebildet ist, daß insbesondere zur Öffnung des Speicherraumes (17) durch Zerstörung einer Speicherwandung mindestens ein in den Speicherraum (17) eindringendes Öffnungsglied (44, 45) vorgesehen ist und daß vorzugsweise der Speicherplatz (9) zur Öffnung des Speicherraumes (17) quer gegenüber dem als Nadelspitze ausgebildeten Öffnungsglied (44, 45) in den Bereitschaftszustand überführbar ist.

8. Spender nach einem der vorhergehenden Ansprüche, dadurch gekennzeichnet, daß mehrere aufeinanderfolgend in den Bereitschaftszustand überführbare, voneinander getrennte Speicherplätze (9) vorgesehen sind, daß insbesondere der Speicherplatz (9) durch eine Drehbewegung in den Bereitschaftszustand überführbar ist, und daß vorzugsweise mehrere Speicherplätze (9) an einem gegenüber dem Grundkörper (2) manuell bewegbaren Magazinkörper (34) vorgesehen sind.

9. Spender nach einem der vorhergehenden Ansprüche, dadurch gekennzeichnet, daß der Grundkörper (2) ein Außengehäuse aus mehreren ineinandergesetzten Gehäuseteilen (3 bis 6) bildet, welche im Querschnitt gemeinsam sowie in Strömungsrichtung aufeinanderfolgend die Fluidführung (14) begrenzen, daß insbesondere ein die Vorlage (21) sowie den Anfang des abfallenden Kanalabschnittes (28) begrenzender zweiter Gehäuseteil (5) an voneinander abgekehrten Seiten schalenförmig ausgebildet und zur Bildung eines Subgehäuses (3, 5) vollständig versenkt in einen den Speicherplatz (9) unmittelbar tragenden ersten Gehäuseteil (3) auf dessen vom Speicherplatz (9) abgekehrten Seite eingesetzt ist und daß vorzugsweise in voneinander abgekehrte Seiten des Subgehäuses (3, 5) einerseits ein den Auslaß (12) aufweisender Gehäuseteil (4) und andererseits ein den Speicherplatz (9) abdeckender Gehäuseteil (6) eingesetzt ist.

10. Spender nach einem der vorhergehenden Ansprüche, dadurch gekennzeichnet, daß der Förderstrom allein durch Atemluft zu erzeugen ist, daß insbesondere der Förderstrom mit einer an dem Grundkörper (2) angeordneten Pumpe (50) zu erzeugen ist und daß vorzugsweise die Pumpe (50) auf der von der Vorlage (21) abgekehrten Seite des Speicherplatzes (9) liegt und ihre Betätigungshandhabe (33) einen den Grundkörper (2) am Außenumfang übergreifenden Enddeckel der Austragvorrichtung (1) bildet.

11. Spender nach einem der vorhergehenden Ansprüche, dadurch gekennzeichnet, daß mindestens ein Bereich (12, 18, 20 bis 29, 44 bis 48, 49) der Fluidführung (14) od.dgl. mit einer Beschichtung versehen ist, daß insbesondere eine Antihaft-Beschichtung vorgesehen ist und daß vorzugsweise im wesentlichen alle mit dem Medium in Berührung gelangenden Bereiche des Spenders 1 mit der Beschichtung versehen sind.
